# EUROPEAN PATENT SPECIFICATION

(11) **EP 1 335 761 B1**
(45) Date of publication and mention of the grant of the patent: **26.09.2007**
(21) Application number: 01973601.6
(22) Date of filing: 28.09.2001
(51) Int. Cl.: A61L 31/10, A61L 27/34

(54) **COATINGS FOR MEDICAL DEVICES**
BESCHICHTUNGEN FÜR MEDIZINISCHE VORRICHTUNGEN
REVETEMENTS DESTINES A DES DISPOSITIFS MEDICAUX

(30) Priority: 29.09.2000 US 675882; 25.09.2001 US 962292
(43) Date of publication of application: 20.08.2003
(73) Proprietor: ETHICON, INC., Somerville, NJ 08876 (US)
(72) Inventor: LLANOS, Gerard, H., Stewartsville, NJ 08886 (US); NARAYANAN, Pallassana, Belle Mead, NJ 08502 (US); ROLLER, Mark, B., North Brunswick, NJ 08902 (US); SCOPELIANOS, Angelo, Whitehouse Station, NJ 08889 (US)
(74) Representative: Mercer, Christopher Paul
(86) International application number: PCT/US2001/030389
(87) International publication number: WO 2002/026280

(56) References cited:
- EP-A- 0 633 032
- WO-A-00/38754
- WO-A-98/08463
- DE-A- 19 723 723
- US-A- 5 759 205

## Description

### FIELD OF THE INVENTION

The invention relates to the use of polyfluoro copolymers as coatings for implantable surgical medical devices.

### BACKGROUND OF THE INVENTION

Implantable medical devices are used in various medical procedures. Such devices include, without limitation, stents, catheters, sutures, meshes, vascular grafts, shunts and filters for removing emboli.

Stents, which generally are open tubular structures, have become increasingly important in medical procedures to restore the function of body lumens. Stents now are commonly used in translumenial procedures such as angioplasty to restore adequate blood flow to the heart and other organs. However, deployment of stents may stimulate foreign body reactions thereto that result in thrombosis or restenosis.

To avoid these complications, a variety of stent coatings and compositions have been proposed to reduce the incidence of these complications. The coatings may be capable themselves of reducing the stimulus the stent provides to the injured lumen wall, thus reducing the tendency towards thrombosis or restenosis. Alternately, the coating may deliver a pharmaceutical/therapeutic agent or drug to the lumen that reduces smooth muscle tissue proliferation or restenosis. The reported mechanism for delivery of the agent has been via diffusion of the agent through either the bulk polymer, or through pores that are created in the polymer structure, or by erosion of a biodegradable coating.

Both bioabsorbable and biostable compositions have been reported as coatings for stents. They generally have been polymeric coatings that either encapsulate a pharmaceutical/ therapeutic agent or drug, e.g. taxol, rapamycin, etc., or bind such an agent to the surface, e.g. heparin-coated stents. These coatings are applied to the stent in a number of ways, including, though not limited to, dip, spray, or spin coating processes.

One class of biostable materials that has been reported as coatings for stents is polyfluoro homopolymers. Polytetrafluoroethylene (PTFE) homopolymers have been used as implants for many years. These homopolymers are not soluble in any solvent at reasonable temperatures and therefore are difficult to coat onto small medical devices while maintaining important features of the devices (e.g. slots in stents).

EP-0633032-A discloses a vascular prosthesis comprising a tubular porous body formed of a synthetic resin and, wound around the body, a tube, fibre or sheet formed from a polymeric material and combined with an antibacterial substance. The polymeric material is polytetrafluoroethylene or a tetrafluoroethylene-hexafluoropropylene copolymer and/or the synthetic resin is polytetrafluoroethylene.

DE19723723-A discloses a polymer coating on a prosthesis, implant or body electrode consists of a vinylidene fluoride/tri- or tetra-fluoroethylene copolymer (optionally as a composite with a polyacrylate, polystyrene and/or polycarbonate) and is characterised by being electrically polarised so as to impart piezoelectric properties.

WO 0038754-A discloses an expandable prosthesis comprising: a self-expanding stent deployable between a substantially radially compressed and expanded configuration, the self-expanding stent including a first and a second end and an outer surface extending therebetween, and a biocompatible coating attached to at least a portion of the outer surface of the self-expanding stent in the radially compressed configuration to inhibit its radial expansion to the radially expanded configuration. The biocompatible coating is expanded polytetrafluoroethylene (ePTFE) or similar fluoropolymer material such as copolymers of tetrafluoroethylene with other monomers, e.g. ethylene, chlorotrifluoroethylene, perfluoroalkoxy-tetrafluoro- ethylene or a fluorinated propylene e.g. hexafluoropropylene.

US-5759205-A discloses biocompatible orthopaedic or dental implant comprising a prosthesis having a tissue-contacting surface of a negatively charged polymeric electret material characterized by a bulk monopolar charge which produces an external electrostatic field to promote bone cell ingrowth and adherence to the prosthesis. The preferred fluoropolymer electret material is fluorinated ethylene propylene copolymer (FEP) but other materials that can be used include polytetrafluoroethylene (PTFE), polyvinylidenefluoride (PVDF) and polyvinylidenefluoride- trifluoroethylene copolymers (P(VDF-TrFE)).

WO 9808463-A discloses a medical device resistant to growth of micro-organisms comprising: an element of the device having external surfaces which will be exposed to an environment containing micro-organisms when placed on or into an animal cavity; and a fluoropolymer capable of inhibiting growth of said micro-organisms comprising said surface. The fluorocarbon polymer is preferably a polymer or copolymer of at least one monomer selected from tetrafluoroethylene, chlortrifluoroethylene, vinylidene fluoride and hexafluoro-propylene and, particularly preferably, a fluoroalkyl substituted siloxane.

Stents with coatings made from polyvinylidenefluoride homopolymers and containing pharmaceutical/therapeutic agents or drugs for release have been suggested. However, like most crystalline polyfluoro homopolymers, they are difficult to apply as high quality films onto surfaces without subjecting them to relatively high temperatures, e.g. greater than about 125-200°C, that correspond to the melting temperature of the polymer.

It would be advantageous to develop coatings for implantable medical devices that will reduce thrombosis, restenosis, or other adverse reactions, that may include, but do not require, the use of pharmaceutical or therapeutic agents or drugs to achieve such affects, and that possess physical and mechanical properties effective for use in such devices when subjected to relatively low maximum temperatures.

### SUMMARY OF THE INVENTION

According to the present invention there is provided an implantable medical device comprising a biocompatible films, as defined in the appendant claims. Medical devices of the invention comprise such coatings and films applied to a surface thereof that is to be in contact with body tissue of a mammal. The biocompatible film provides an inert surface to be in contact with body tissue of a mammal upon implantation of the device in the mammal.

### BRIEF DESCRIPTION OF THE FIGURES

Figure 1 indicates the fraction of drug released as a function of time from coatings used in the present invention over which no topcoat has been disposed.
Figure 2 indicates the fraction of drug released as a function of time from coatings used in the present invention including a topcoat disposed thereon.
Figure 3 indicates the fraction of drug released as a function of time from coatings used in the present invention over which no topcoat has been disposed.
Figure 4 indicates in vivo stent release kinetics of rapamycin from poly(VDF/HFP).

### DETAILED DESCRIPTION OF THE INVENTION

The present invention provides implantable medical devices, e.g. stents, coated with a film of the polyfluoro polymeric coating in amounts effective to reduce thrombosis and/or restenoais when such stents are used in, e.g. angioplasty procedures.

In certain embodiments, the invention provides medical devices that are implanted into the body of a mammal and later retrieved therefrom comprising an inert, low surface energy coating. The low surface energy coating makes wetting of the device surface and protein deposition thereon difficult, which could prolong the time for encapsulation in the body, after which time the device could be removed easily.

In certain embodiments of the invention, although not necessary, the coatings may comprise pharmaceutical or therapeutic agents in amounts effective for achieving desired purposes, e.g. for reducing thrombosis or restenosis, and stents coated with such coatings may provide sustained release of the agents. Films prepared from certain polyfluoro copolymer coatings of the present invention provide the physical and mechanical properties required of conventional coated medical devices, even where maximum temperatures to which the device, coatings and films are exposed are limited to relatively low temperatures, e.g. less than 100°C, preferably at about ambient temperatures. This is particularly important when using the coating/film to deliver pharmaceutical/therapeutic agent or drugs that are heat sensitive, or when applying the coating onto temperature-sensitive devices such as, but not limited to, catheters. When maximum exposure temperature is not an issue, e.g. where heat-stable agents such as itraconazole are incorporated into the coatings, higher melting thermoplastic polyfluoro copolymers may be used and, if very high elongation and adhesion is required, elastomers may be used. If desired or required, the polyfluoro elastomers may be crosslinked by standard methods described in, e.g. Modern Fluoropolymers, J. Shires editor, John Wiley & Sons, New York, 1997, pp. 77-87.

The present invention employs polyfluoro copolymers that provide improved biocompatible coatings for medical devices. These coatings provide inert surfaces to be in contact with body tissue of a mammal, e.g. a human, sufficient to reduce thrombosis, or restenosis, or other undesirable reactions. While most reported coatings made from polyfluoro homopolymers are insoluble and/or require high heat, e.g. greater than 125°C, to obtain films with adequate physical and mechanical properties for use on implantable devices, e.g. stents, or are not particularly tough or elastomeric, films prepared from the polyfluoro copolymer coatings of the present invention provide adequate adhesion, toughness or elasticity, and resistance to cracking when formed on medical devices claimed herein. In certain embodiments, this is the case even where the coated devices are subjected to relatively low maximum temperatures, e.g. less than 100°C, preferably less than 65°C, and more preferably 60°C or less. In such cases, preferred polyfluoro copolymers may comprise the polymerized residue of from 65 to 55 weight percent polymerized residue of the first moiety, i.e. VDF, and from 35 to 45 weight percent polymerized residue of the second moiety, i.e. hexafluoropropylene. In certain embodiments, such polyfluoro copolymers will be crystalline, although amorphous copolymers of similar composition also are employed.

The polyfluoro copolymers used for coatings employed in the present invention must be film-forming polymers that have molecular weight high enough so as not to be waxy or tacky. The polymers and films formed therefrom must adhere to the stent and not be readily deformable after deposition on the stent as to be able to be displaced by hemodynamic stresses. The polymer molecular weight must be high enough to provide sufficient toughness so that films comprising the polymers will not be rubbed off during handling or deployment of the stent. In certain embodiments the coating will not crack where expansion of the stent or other medical devices, such as vena cava filters, occurs. The flow point of the polymer used in the present invention should be above 40°C, preferably above 45°C, more preferably above 50°C and most preferably above 55°C.

Polyfluoro copolymers used in the present invention comprise vinylidinefluoride copolymerized with HFP, in the weight ratio of from 50 to 92 weight percent vinylidinefluoride to 50 to 8 weight percent HFP. Preferably, polyfluoro copolymers used in the present invention comprise from 50 to 85 weight percent VDF copolymerized with from 50 to 15 weight percent HFP. More preferably, the polyfluoro copolymers will comprise from 55 to 70 weight percent VDF copolymerized with from 45 to 30 weight percent HFP. Even more preferably, polyfluoro copolymers comprise from 55 to 65 weight percent VDF copolymerized with from 45 to 35 weight percent HFP. Such polyfluoro copolymers are soluble, in varying degrees, in solvents such as dimethylacetamide (DMAc), tetrahydrofuran, dimethyl formamide, dimethyl sulfoxide and n-methyl pyrrolidone. Some are soluble in methylethylketone (MEK), acetone, methanol and other solvents commonly used in applying coatings to conventional implantable medical devices.

Conventional polyfluoro homopolymers are crystalline and difficult to apply as high quality films onto metal surfaces without exposing the coatings to relatively high temperatures that correspond to the melting temperature (Tm) of the polymer. The elevated temperature serves to provide films prepared from such PVDF homopolymer coatings that exhibit sufficient adhesion of the film to the device, while preferably maintaining sufficient flexability to resist film cracking upon expansion/contraction of the coated medical device. Certain films and coatings according to the present invention provide these same physical and mechanical properties, or essentially the same properties, even when the maximum temperatures to which the coatings and films are exposed is less than 100°C, and preferably less than 65°C. This is particularly important when the coatings/films comprise pharmaceutical or therapeutic agents or drugs that are heat sensitive, e.g. subject to chemical or physical degradation or other heat-induced negative affects, or when coating heat sensitive substrates of medical devices, e.g. subject to heat-induced compositional or structural degradation.

Depending on the particular device upon which the coatings and films of the present invention are to be applied and the particular use/result required of the device, polyfluoro copolymers used to prepare such devices may be crystalline, semi-crystalline or amorphous.

Where devices have no restrictions or limitations with respect to exposure of same to elevated temperatures, e.g. 100°C or higher, crystalline polyfluoro copolymers may be employed. Crystalline polyfluoro copolymers tend to resist the tendency to flow under applied stress or gravity when exposed to temperatures above their glass transition (Tg) temperatures. Crystalline polyfluoro copolymers provide tougher coatings and films than their fully amorphous counterparts. In addition, crystalline polymers are more lubricious and more easily handled through crimping and transfer processes used to mount self-expanding stents, e.g. nitinol stents.

Semi-crystalline and amorphous polyfluoro copolymers are advantageous where exposure to elevated temperatures is an issue, e.g. where heat-sensitive pharmaceutical or therapeutic agents are incorporated into the coatings and films, or where device design, structure and/or use preclude exposure to such elevated temperatures. Semi-crystalline polyfluoro copolymer elastomers comprising relatively high levels, e.g. from 30 to 45 weight percent of the HFP, copolymerized with the VDF, have the advantage of reduced coefficient of friction and self-blocking relative to amorphous polyfluoro copolymer elastomers. Such characteristics can be of significant value when processing, packaging and delivering medical devices coated with such polyfluoro copolymers. In addition, such polyfluoro copolymer elastomers comprising such relatively high content of the second moiety serves to control the solubility of certain agents, e.g. Sirolimus, in the polymer and therefore controls permeability of the agent through the matrix.

Polyfluoro copolymers utilized in the present inventions may be prepared by various known polymerization methods. For example, high pressure, free-radical, semi-continuous emulsion polymerization techniques such as those disclosed in Fluoroelastomers-denendence of relaxation phenomena on composition, POLYMER 30, 2180, 1989, by Ajroldi, *et al*, may be employed to prepare amorphous polyfluoro copolymers, some of which may be elastomers. In addition, free-radical batch emulsion polymerization techniques disclosed herein may be used to obtain polymers that are semi-crystalline, even where relatively high levels of the second moiety, e.g. greater than 19-20 mole percent (equivalent to 36-37 weight percent), are included.

One embodiment of the invention comprises stents coated with a film of a polyfluoro copolymer according to the present invention. Conventional stents are used in translumenial procedures such as angioplasty to restore adequate blood flow to the heart and other organs. They generally are cylindrical and perforated with passages that are slots, ovoid, circular or the like shape. Stents also may be composed of helically wound or serpentine wire structures in which the spaces between the wires form passages. Stents may be flat perforated structures that are subsequently rolled to form tubular or cylindrical structures that are woven, wrapped, drilled, etched or cut to form passages. Examples of stents that may be advantageously coated by polyfluoro copolymers of the present invention include, but are not limited to, stents described in U.S. Patent Nos. 4,733,665; 4,800,882; 4,886,062, 5,514,154, and 6,190,403. These stents can be made of biocompatible materials, including biostable and bioabsorbable materials. Suitable biocompatible metals include, but are not limited to, stainless steel, tantalum, titanium alloys (including nitinol), and cobalt alloys (including cobalt-chromium-nickel alloys). Suitable nonmetallic biocompatible materials include, but are not limited to, polyamides, polyolefins (i.e. polypropylene, polyethylene etc.), nonabsorbable polyesters (i.e. polyethylene terephthalate), and bioabsorbable aliphatic polyesters (i.e. homopolymers and copolymers of lactic acid, glycolic acid, lactide, glycolide, para-dioxanone, trimethylene carbonate, ε-caprolactone, and blends thereof).

The film-forming biocompatible polymer coatings generally are applied to the stent in order to reduce local turbulence in blood flow through the stent, as well as adverse tissue reactions. The coatings and films formed therefrom also may be used to administer a pharmaceutically active material to the site of the stent placement. Generally, the amount of polymer coating to be applied to the stent will vary depending on, among other possible parameters, the particular polyfluoro copolymer used to prepare the coating, the stent design and the desired effect of the coating. Generally, the coated stent will comprise from 0.1 to 15 weight percent of the coating, preferably from 0.4 to 10 weight percent. The polyfluoro copolymer coatings may be applied in one or more coating steps, depending on the amount of polyfluoro copolymer to be applied. Different polyfluoro copolymers may be used for different layers in the stent coating. In fact, in certain embodiments, it is highly advantageous to use a diluted first coating solution comprising a polyfluoro copolymer as a primer to promote adhesion of a subsequent polyfluoro copolymer coating layer that may contain pharmaceutically active materials. The individual coatings may be prepared from different polyfluoro copolymers.

Additionally, a top coating can be applied to delay release of the pharmaceutical agent, or they could be used as the matrix for the delivery of a different pharmaceutically active material. Layering of coatings can be used to stage release of the drug or to control release of different agents placed in different layers.

Blends of polyfluoro copolymers also may be used to control the release rate of different agents or to provide desirable balance of coating properties, i.e. elasticity, toughness, etc., and drug delivery characteristics, e.g. release profile. Polyfluoro copolymers with different solubilities in solvents can be used to build up different polymer layers that may be used to deliver different drugs or to control the release profile of a drug. For example, polyfluoro copolymers comprising 85.5/14.5 (wt/wt) of poly(VDF/HFP) and 60.6/39.4 (wt/wt) of poly(VDF /HFP) are both soluble in DMAc. However, only the 60.6/39.4 poly(VDF/HFP) polyfluoro copolymer is soluble in methanol.

So, a first layer of the 85.5/14.5 poly(VDF/HFP) polyfluoro copolymer comprising a drug could be overcoated with a topcoat of the 60.6/39.4 poly(VDF/HFP) polyfluoro copolymer made with the methanol solvent. The top coating can be used to delay the drug deliver of the drug contained in the first layer. Alternatively, the second layer could contain a different drug to provide for sequential drug delivery. Multiple layers of different drugs could be provided by alternating layers of first one polyfluoro copolymer, then the other. As will be readily appreciated by those skilled in the art numerous layering approaches can be used to provide the desired drug delivery.

The coatings can be used to deliver therapeutic and pharmaceutic agents such as, but not limited to: antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which don't have the capacity to synthesize their own asparagine); antiproliferative/antimitotic alkylating agents such as nitrogen mustards(mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin),trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine{cladribine}); platinum coordination complexes (cisplatin, carboplatin), procarbazine, hydroxyurea, mitotane, aminoglutethimide; hormones (i.e.estrogen); Anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; Indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); Angiogenic agents: vascular endothelial growth factor (VEGF), fibroblast growth factor (FGF); nitric oxide donors; cell cycle inhibitors; mTOR inhibitors; growth factor signal transduction knase inhibitors; antisense oligonucleotide; prodrug molecules; and combinations thereof.

Coatings may be formulated by mixing one or more therapeutic agents with the coating polyfluoro copolymers in a coating mixture. The therapeutic agent may be present as a liquid, a finely divided solid, or any other appropriate physical form. Optionally, the coating mixture may include one or more additives, e.g., nontoxic auxiliary substances such as diluents, carriers, excipients, stabilizers or the like. Other suitable additives may be formulated with the polymer and pharmaceutically active agent or compound. For example, a hydrophilic polymer may be added to a biocompatible hydrophobic coating to modify the release profile, or a hydrophobic polymer may be added to a hydrophilic coating to modify the release profile. One example would be adding a hydrophilic polymer selected from the group consisting of polyethylene oxide, polyvinyl pyrrolidone, polyethylene glycol, carboxylmethyl cellulose, and hydroxymethyl cellulose to a polyfluoro copolymer coating to modify the release profile. Appropriate relative amounts can be determined by monitoring the in vitro and/or *in vivo* release profiles for the therapeutic agents.

The best conditions for the coating application are when the polyfluoro copolymer and pharmaceutic agent have a common solvent. This provides a wet coating that is a true solution. Less desirable, yet still usable, are coatings that contain the pharmaceutical agent as a solid dispersion in a solution of the polymer in solvent. Under the dispersion conditions, care must be taken to ensure that the particle size of the dispersed pharmaceutical powder, both the primary powder size and its aggregates and agglomerates, is small enough not to cause an irregular coating surface or to clog the slots of the stent that need to remain essentially free of coating. In cases where a dispersion is applied to the stent and the smoothness of the coating film surface requires improvement, or to be ensured that all particles of the drug are fully encapsulated in the polymer, or in cases where the release rate of the drug is to be slowed, a clear (polyfluoro copolymer only) topcoat of the same polyfluoro copolymer used to provide sustained release of the drug or another polyfluoro copolymer that further restricts the diffusion of the drug out of the coating can be applied. The topcoat can be applied by dip coating with mandrel to clear the slots, referred to herein as the dip and wipe method. This method is disclosed in United States Patent 6,153,252. Other methods for applying the topcoat include spin coating and spray coating. Dip coating of the top coat can be problematic if the drug is very soluble in the coating solvent, which swells the polyfluoro copolymer, and the clear coating solution acts as a zero concentration sink and redissolves previously deposited drug. The time spent in the dip bath may need to be limited so that the drug is not extracted out into the drug-free bath. Drying should be rapid so that the previously deposited drug does not completely diffuse into the topcoat.

The amount of therapeutic agent will be dependent upon the particular drug employed and medical condition being treated. Typically, the amount of drug represents 0.001% to 70%, more typically 0.001% to 60%.

The quantity and type of polyfluoro copolymers employed in the coating film containing the pharmaceutic agent will vary depending on the release profile desired and the amount of drug employed. The product may contain blends of the polyfluoro copolymers having different molecular weights to provide the desired release profile or consistency to a given formulation.

Polyfluoro copolymers may release dispersed drug by diffusion. This can result in prolonged delivery (over, say 1 to 2,000 hours, preferably 2 to 800 hours) of effective amounts (say, 0.001 µg/cm²-min to 100 µg/cm²-min) of the drug. The dosage can be tailored to the subject being treated, the severity of the affliction, the judgment of the prescribing physician, and the like. Individual formulations of drugs and polyfluoro copolymers may be tested in appropriate *in vitro* and *in vivo* models to achieve the desired drug release profiles. For example, a drug could be formulated with a polyfluoro copolymer, or blend of polyfluoro copolymers, coated onto a stent and placed in an agitated or circulating fluid system, e.g. 25% ethanol in water. Samples of the circulating fluid could be taken to determine the release profile (such as by HPLC, UV analysis or use of radiotagged molecules). The release of a pharmaceutical compound from a stent coating into the interior wall of a lumen could be modeled in appropriate animal system. The drug release profile could then be monitored by appropriate means such as, by taking samples at specific times and assaying the samples for drug concentration (using HPLC to detect drug concentration). Thrombus formation can be modeled in animal models using the ¹¹¹ In-platelet imaging methods described by Hanson and Harker, Proc. Natl. Acad. Sci. USA 85:3184-3188 (1988). Following this or similar procedures, those skilled in the art will be able to formulate a variety of stent coating formulations.

While not a requirement of the present invention, the coatings and films may be crosslinked once applied to the medical devices. Crosslinking may be affected by any of the known crosslinking mechanisms, such as chemical, heat or light. In addition, crosslinking initiators and promoters may be used where applicable and appropriate. In those embodiments utilizing crosslinked films comprising pharmaceutical agents, curing may affect the rate at which the drug diffuses from the coating. Crosslinked polyfluoro copolymers films and coatings of the present invention also may be used without drug to modify the surface of implantable medical devices.

### Examples:

### Example 1:

A poly(VDF) homopolymer (Solef 1008 from Solvay Advanced Polymers, Houston, TX, Tm about 175°C) and polyfluoro copolymers of poly(VDF/HFP), 92/8 and 91/9 weight percent VDF/HFP, respectively, as determined by F¹⁹ NMR (*eg:* Solef 11010 and 11008, Solvay Advanced Polymers, Houston, TX, Tm about 159°C and 160°C, respectively) were examined as potential coatings for stents. These polymers are soluble in solvents such as, but not limited to, DMAc, N,N-dimethylformamide (DMF), dimethyl sulfoxide (DMSO), N-methylpyrrolidone (NMP), tetrahydrofuran (THF) and acetone. Polymer coatings were prepared by dissolving the polymers in acetone, at 5 weight percent as a primer, or by dissolving the polymer in 50/50 DMAc/acetone, at 30 weight percent as a topcoat. Coatings that were applied to the stents by dipping and dried at 60°C in air for several hours, followed by 60°C for 3 hours in a <100 mm Hg vacuum, resulted in white foamy films. As applied, these films adhered poorly to the stent and flaked off, indicating they were too brittle. When stents coated in this manner were heated above 175°C, i.e. above the melting temperature of the polymer, a clear, adherent film was formed. Such coatings require high temperatures, e.g. above the melting temperature of the polymer, to achieve high quality films.

### Example 2:

A polyfluoro copolymer (Solef 21508) comprising 85.5 weight percent VDF copolymerized with 14.5 weight percent HFP, as determined by F¹⁹ NMR, was evaluated. This copolymer is less crystalline than the polyfluoro homopolymer and copolymers described in Example 1. It also has a lower melting point reported to be about 133°C. Once again, a coating comprising about 20 weight percent of the polyfluoro copolymer was applied from a polymer solution in 50/50 DMAc/MEK. After drying (in air) at 60°C for several hours, followed by 60°C for 3 hours in a <100 mtorr Hg vacuum, clear adherent films were obtained. This eliminated the need for a high temperature heat treatment to achieve high quality films. Coatings were smoother and more adherent than those of Example 1. Some coated stents that underwent expansion show some degree of adhesion loss and "tenting" as the film pulls away from the metal. Where necessary, modification of coatings containing such copolymers may be made, e.g. by addition of plasticizers or the like to the coating compositions. Films prepared from such coatings may be used to coat stents or other medical devices, particularly where those devices are not susceptible to expansion to the degree of the stents.

The coating process above was repeated, this time with a coating comprising the 85.5/14.6 (wt/wt) (VDF/HFP) and about thirty (30) weight percent of rapamycin (Wyeth-Ayerst Laboratories, Philadelphia, PA), based on total weight of coating solids. Clear films that would occasionally crack or peel upon expansion of the coated stents resulted. It is believed that inclusion of plasticizers and the like in the coating composition will result in coatings and films for use on stents and other medical devices that are not susceptible to such cracking and peeling.

### Example 3:

Polyfluoro copolymers of still higher HFP content then were examined. This series of polymers were not semi-crystalline, but rather are marketed as elastomers. One such copolymer is Fluorel FC-2261Q (from Dyneon, a 3M-Hoechst Enterprise, Oakdale, MN), a 60.6/39.4 (wt/wt) copolymer of VDF/HFP. Although this copolymer has a Tg well below room temperature (Tg about -20°C), it is not tacky at room temperature or even at 60°C. This polymer has no detectable crystallinity when measured by Differential Scanning Calorimetry (DSC) or by wide angle X-ray diffraction. Films formed on stents as described above were non-tacky, clear, and expanded without incident when the stents were expanded.

The coating process above was repeated, this time with coatings comprising the 60.6/39.4 (wt/wt) poly(VDF/HFP) and about nine (9), thirty (30) and fifty (50) weight percent of rapamycin , based on total weight of coating solids, respectively. Coatings comprising about 9 and 30 weight percent rapamycin provided white, adherent, tough films that expanded without incident on the stent. Inclusion of 50% drug, in the same manner, resulted in some loss of adhesion upon expansion.

Changes in the comonomer composition of the polyfluoro copolymer also can affect the nature of the solid state coating, once dried. For example, the semi-crystalline copolymer, Solef 21508, containing 85.5% VDF polymerized with 14.5% by weight HFP forms homogeneous solutions with about 30% rapamycin (drug weight divided by total solids weight, e.g. drug plus copolymer) in DMAc and 50/50 DMAc/MEK. When the film is dried (60°C/16 hours followed by 60°C/3 hours in vacuum of 100 mm Hg) a clear coating, indicating a solid solution of the drug in the polymer, is obtained. Conversely, when an amorphous copolymer, Fluorel FC-2261Q, of poly(VDF/HFP) at 60.6/39.5 (wt/wt) forms a similar 30% solution of rapamycin in DMAc/MEK and is similarly dried, a white film, indicating phase separation of the drug and the polymer, is obtained. This second drug containing film is much slower to release the drug into an in vitro test solution of 25% ethanol in water than is the former clear film of crystalline Solef 21508. X-ray analysis of both films indicates that the drug is present in a non-crystalline form. Poor or very low solubility of the drug in the high HFP-containing copolymer results in slow permeation of the drug through the thin coating film. Permeability is the product of diffusion rate of the diffusing species (in this case the drug) through the film (the copolymer) and the solubility of the drug in the film.

### Example 4: In vitro release results of rapamycin from coating.

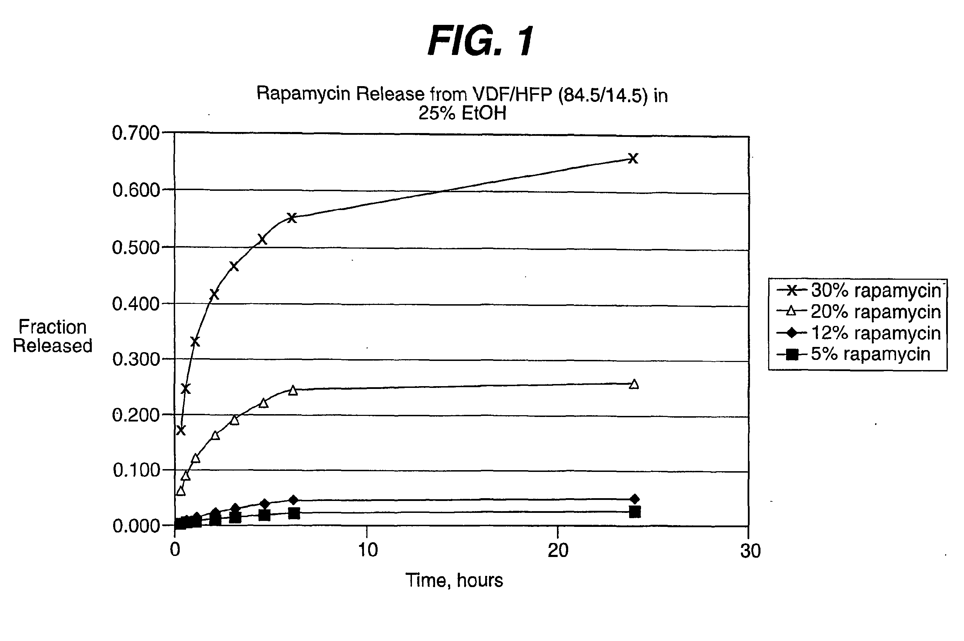

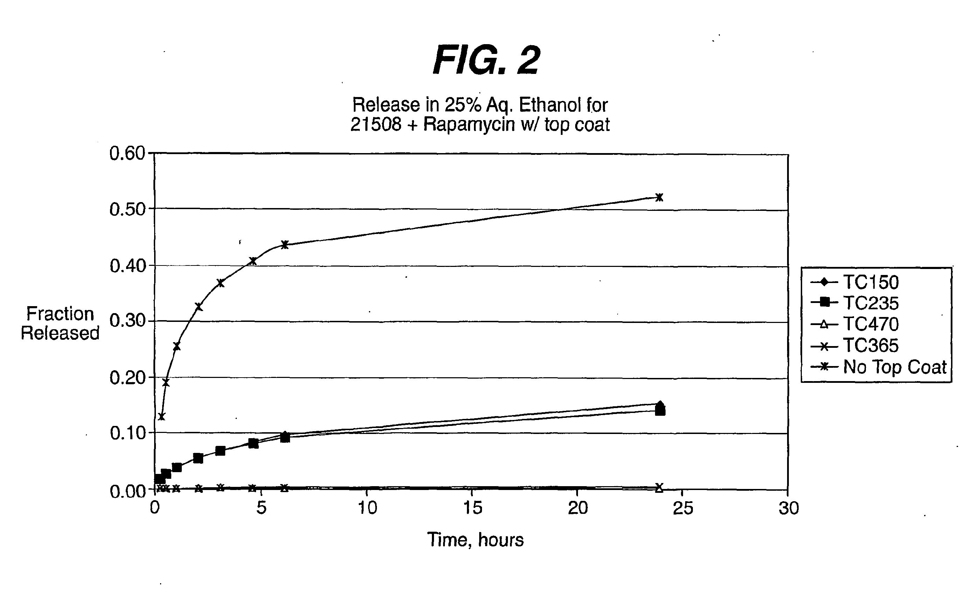

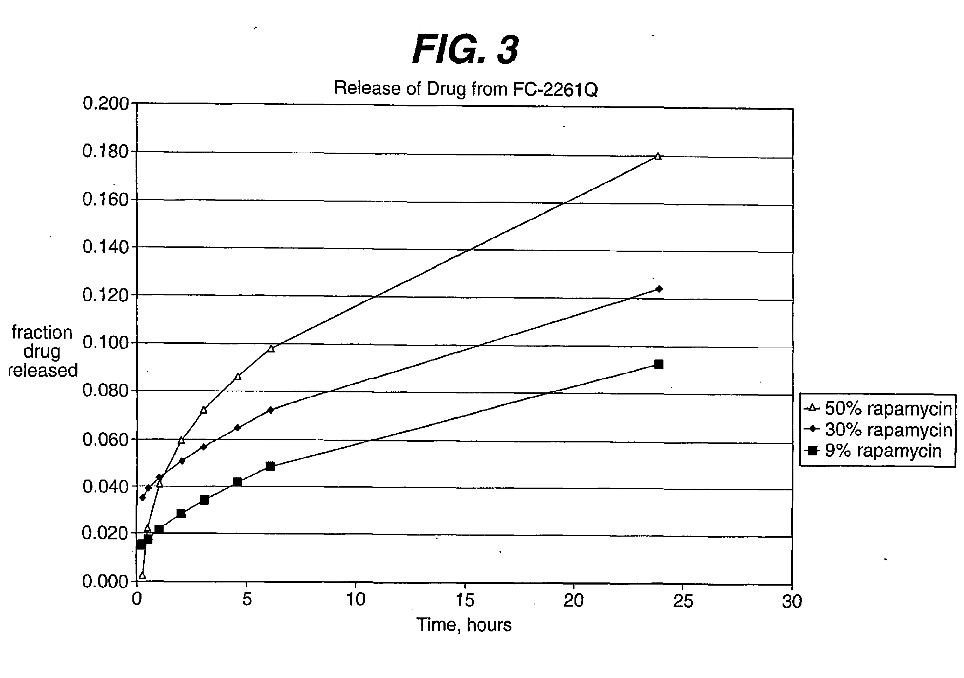

Figure 1 is a plot of data for the 85.5/14.5 VDF/HFP polyfluoro copolymer, indicating fraction of drug released as a function of time, with no topcoat. Figure 2 is a plot of data for the same polyfluoro copolymer over which a topcoat has been disposed, indicating that most effect on release rate is with a clear topcoat. As shown therein, TC150 refers to a device comprising 150 micrograms of topcoat, TC235 refers to 235 micrograms of topcoat, etc. The stents before top coating had an average of 750 micrograms of coating containing 30% rapamycin ( based on drug/[drug + polymer]) Figure 3 is a plot for the 60.6/39.4 VDF/HFP polyfluoro copolymer, indicating fraction of drug released as a function of time, showing significant control of release rate from the coating without the use of a topcoat. Release is controlled by loading of drug in the film.

### Example 5: in vivo stent release kinetics of rapamycin from poly (VDF/HFP).

Nine (9) New Zealand white rabbits (2.5-3.0 kg) on a normal diet were given aspirin 24 hours prior to surgery, again just prior to surgery and for the remainder of the study. At the time of surgery, animals were premedicated with Acepromazine (0.1-0.2 mg/kg) and anesthetized with a Ketamine/Xylazine mixture (40 mg/kg and 5 mg/kg, respectively). Animals were given a single intraprocedural dose of heparin (150 IU/kg, i.v.)

Arteriectomy of the right common carotid artery was performed and 5 F catheter introducer (Cordis, Inc.) placed in the vessel and anchored with ligatures. Iodine contrast agent was injected to visualize the right common carotid artery, brachlocephalic trunk and aortic arch. A steerable guide wire (0.014 inch/180 cm, Cordis, Inc.) was inserted via the introducer and advanced sequentially into each iliac artery to a location where the artery possesses a diameter closest to 2 mm using the angiographic mapping done previously. Two stents coated with a film made from poly(VDF/HFP):(60.6/39.4), with about 30% rapamycin( based on drug/[drug + polymer]) were deployed in each animal where feasible, one in each iliac artery, using 3.0 mm balloon and inflation to 8-10 ATM for 30 seconds followed after a 1 minute interval by a second inflation to 8-10 ATM for 30 seconds. Follow-up angiographs visualizing both iliac arteries are obtained to confirm correct deployment position of the stent.

At the end of procedure, the carotid artery was ligated and the skin is closed with 3/0 vicryl suture using a one layered interrupted closure. Animals were given butoropanol (0.4 mg/kg, s.c.) and gentamycin (4 mg/kg, i.m.). Following recovery, the animals were returned to their cages and allowed free access to food and water.

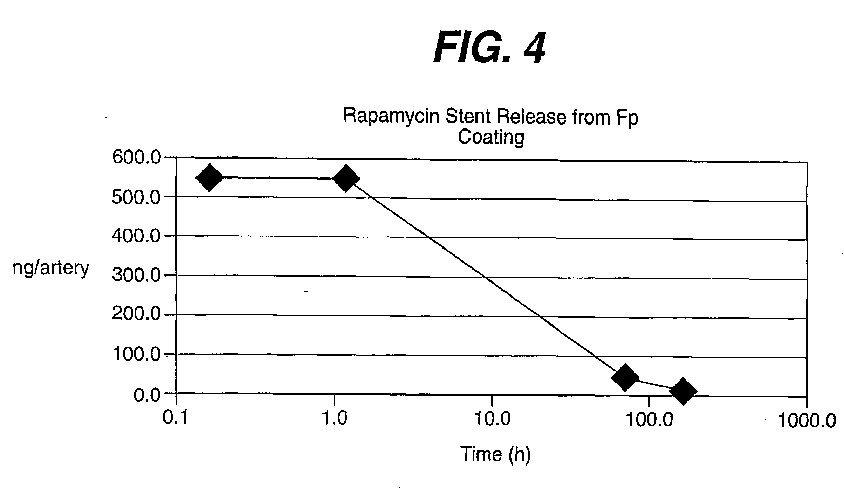

Due to early deaths and surgical difficulties, 2 animals were not used in this analysis. Stented vessels were removed from the remaining 7 animals at the following time points: 1 vessel (1 animal) at 10 min post implant; 6 vessels (3 animals) between 45 min and 2 h post-implant (average, 1.2 hours); 2 vessels (2 animals) at 3 d post implant; and 2 vessels (1 animal) at 7 d post-implant. In one animal at 2 hours, the stent was retrieved from the aorta rather than the iliac artery. Upon removal, arteries were carefully trimmed at both the proximal and distal ends of the stent. Vessels were then carefully dissected free of the stent, flushed to remove any residual blood, and both stent and vessel frozen immediately, wrapped separately in foil, labeled and kept frozen at -80 °C. When all samples had been collected, vessels and stents were frozen, transported and subsequently analyzed for rapamycin in tissue. Results are shown in Figure 4.

### Example 6: Purifying the polymer.

The Fluorel FC-2261Q copolymer was dissolved in MEK at about 10 weight percent and was washed in a 50/50 mixture of ethanol/water. The (ethanol/water): MEK solution ratio = about 14:1. The polymer precipitated out and was separated from the solvent phase by centrifugation. The polymer again was dissolved in MEK and the washing procedure repeated. The polymer was dried after each washing step at 60°C in a vacuum oven (<200 mtorr) over night.

### Example 7: In vivo testing of coated stents in porcine coronary arteries.

CrossFlex® stents (available from Cordis, a Johnson & Johnson Company) were coated with the "as received" Fluorel FC-2261Q PVDF copolymer and with the purified polyfluoro copolymer of example 6, using the dip and wipe approach. The coated stents were sterilized using ethylene oxide and a standard cycle. The coated stents and bare metal stents (controls) were implanted in porcine coronary arteries, where they remained for 28 days.

Angiography was performed on the pigs at implantation and at 28 days. Angiography indicated that the control uncoated stent exhibited about 21 percent restenosis. The polyfluoro copolymer "as received" exhibited about 26% restenosis (equivalent to the control) and the washed copolymer exhibited about 12.5% restenosis.

Histology results reported neointimal area at 28 days to be 2.89±0.2, 3.57±0.4 and 2.75±0.3, respectively, for the bare metal control, the unpurified copolymer and the purified copolymer.

### Example 8:

Utilizing the following high pressure, free-radical batch emulsion polymerization technique, a series of semi-crystalline, poly(VDF/HFP) copolymer elastomers was prepared.

The VDF and HFP monomers were premixed under pressure in a pressure vessel. HPLC-grade water, surfactant and initiator were mixed outside of a 2 liter Zipperclave® reactor (Autoclave Engineers, Erie, PA) and then charged to the reactor, which then was sealed. The premixed monomers then were transferred under nitrogen pressure to the reactor. While stirring, the reactor was raised to the desired temperature and held for a predetermined period of time. The reactor then was cooled and residual monomer vented. The resultant polymer latex was removed from the reactor and coagulated or crashed by adding dilute hydrochloric acid, followed by aqueous sodium chloride. The resulting polymer was washed extensively with water and dried.

The polyfluoro copolymers then were compared with respect to kinetic coefficient of friction of a film prepared therefrom to the kinetic coefficient of friction of a film prepared from a commercial amorphous polyfluoro copolymer comprising 59.5 weight percent VDF copolymerized with 40.5 weight percent HFP utilizing the following procedure.

A 57.2 mm wide by 140.0 mm long polymer film was cast on a 101.6 mm wide by 203.2 mm long aluminum panel (Q-panel, anodized finish, A-48). A silicone rubber gasket was placed on the aluminum panel and clamped using binder clips. The mold was leveled in a fume hood using a bubble level. Approximate 5.0 g of 10.0% polymer solution in methyl ethyl ketone was poured into the mold slowly. The film was dried at room temperature for 3 days followed by 3 hours at 23°C and 50% R.H. prior to testing.

The kinetic coefficient of friction of the polymer film was measured in accordance with the method described in ASTM D 1894 -00, "Static and Kinetic Coefficients of Friction of Plastic Film and Sheeting", Method C. A 46.5 g Teflon block, 25.4 mm wide by 41.3 mm long by 19.1 mm thick, with an eye screw fastened in one end was used as a sled. The surface of the sled that contacted to the film was polished using 500-grit sandpaper. The Teflon sled was attached to a flexible beaded chain and pulled using an Instron tensile tester at a rate of 150 mm/min., at 23°C and 50% R.H. Five measurements was made on each film sample. The thickness of the film was measured using a digital thickness gauge. The kinetic coefficient test results are given in Table I. The maximum kinetic coefficient of friction of five measurements of each film were averaged and reported.

The Differential Scanning Calorimetry (DSC) data were obtained on the following polymers using vacuum dried films in a TA Instruments Model 2920 Modulated DSC in standard (non-modulated) DSC mode. The samples were quenched to -80°C and heated at 10°C/min to 275°C in nitrogen. The data are reported as ΔH (J/g) for endothermic, melting events above glass transition temperature (Tg).

**Table I**

| Kinetic Coefficient of Polymer Film | | | |
|---|---|---|---|
| **Sample I.D. Wt/wt VDF/HFP** | **Film Thickness (µm)** | **Max. Kinetic Coefficient** | **DSC ΔH (J/g)** |
| Commercial | 22.9 | 2.65 | None |
| 59.5/40.5 | | σ = 0.17 | |
| Polymer 8a | 38.6 | 1.71 | 16.5 |
| 55.1/44.9 | | σ = 0.09 | |
| Polymer 8b | 27.5 | 0.27 | 15 |
| 56.8/43.2 | | σ = 0.03 | |
| Polymer 8c | 25.4 | 0.35 | 19.5 |
| 68.3/31.7 | | σ = 0.07 | |
| Polymer 8d | 21.1 | 2.12 | 4.5 |
| 59.9/40.1 | | σ = 0.04 | |

## Claims

1. An implantable medical device comprising:
a biocompatible film effective to provide an inert surface to be in contact with body tissue of a mammal upon implantation of said device in said mammal,
said film comprising a polyfluoro copolymer comprising:
from greater than 85 to 92 weight percent of polymerized residue of a vinylidenefluoride; and
from less than 15 to 8 weight percent of polymerized residue of hexafluoropropylene.

2. The device of claim 1, wherein said film further comprises an effective amount of a therapeutic and/or pharmaceutical agent.

3. The device claim 1 or 2, wherein said polyfluoro copolymer is effective to provide said film with properties effective for use in coating said implantable medical device when said coated device is subjected to a maximum temperature of less than 100°C.

## Patentansprüche

1. Implantierbare medizinische Vorrichtung, welche umfaßt:
einen biologisch kompatiblen Film, der darin wirksam ist, eine inerte Oberfläche bereitzustellen, um in Kontakt mit Körpergewebe eines Säugers bei Implantation besagter Vorrichtung in besagten Säuger zu kommen,
wobei besagter Film ein Polyfluorcopolymer umfaßt, welches umfaßt:
von mehr als 85 bis 92 Gewichtsprozent polymerisierter Rest eines Vinylidenfluorids; und
von weniger als 15 bis 8 Gewichtsprozent polymerisierter Rest von Hexafluorpropylen.

2. Vorrichtung nach Anspruch 1, **dadurch gekennzeichnet, daß** besagter Film weiter eine wirksame Menge eines therapeutischen und/oder pharmazeutischen Mittels umfaßt.

3. Vorrichtung nach Anspruch 1 oder 2, **dadurch gekennzeichnet, daß** besagtes Polyfluorcopolymer darin wirksam ist, besagten Film mit Eigenschaften zu versehen, die wirksam sind zur Verwendung bei der Beschichtung besagter implantierbarer medizinischer Vorrichtung, wenn besagte beschichtete Vorrichtung einer maximalen Temperatur von weniger als 100°C unterworfen wird.

## Revendications

1. Dispositif médical implantable, comprenant :
un film biocompatible efficace pour constituer une surface inerte destinée à être en contact avec un tissu du corps d'un mammifère après l'implantation dudit dispositif chez ledit mammifère,
ledit film comprenant un copolymère polyfluoré qui comprend :
plus de 85% jusqu'à 92% en poids d'un résidu de fluorure de vinylidène polymérisé ; et
moins de 15% jusqu'à 8% en poids d'un résidu d'hexafluoropropylène polymérisé.

2. Dispositif selon la revendication 1, dans lequel ledit film comprend en plus une quantité efficace d'un agent thérapeutique et/ou pharmaceutique.

3. Dispositif selon la revendication 1 ou la revendication 2, dans lequel ledit copolymère polyfluoré est efficace pour conférer audit film des propriétés efficaces en vue de l'utiliser pour revêtir ledit dispositif médical implantable lorsque ledit dispositif revêtu est exposé à une température maximale inférieure à 100°C.
